# FASCICULE DE BREVET EUROPEEN

(11) **EP 2 240 013 B1**
(45) Date de publication et mention de la délivrance du brevet: **31.08.2016**
(21) Numéro de dépôt: 09707168.2
(22) Date de dépôt: 05.02.2009
(51) Int. Cl.: A01N 25/02, A01N 33/02, A01N 33/12, A01P 1/00

(54) **UTILISATION D'UNE COMPOSITION POUR LE TRAITEMENT DES SURFACES DES BATIMENTS D'ELEVAGE ET/OU DE LEUR MATERIEL**
VERWENDUNG EINER ZUSAMMENSETZUNG ZUR BEARBEITUNG DER OBERFLÄCHEN VON NUTZTIERBEHÄLTERN ODER DES DARIN ENTHALTENEN MATERIALS
USE OF A COMPOSITION FOR TREATING THE SURFACES OF LIVESTOCK BUILDINGS AND/OR THE MATERIAL THEREIN

(30) Priorité: 05.02.2008 FR 0800577
(43) Date de publication de la demande: 20.10.2010
(73) Titulaire: CID LINES N.V., 8900 Ieper (BE)
(72) Inventeur: ALASRI, Richard, 8900 Ieper (BE); BRUTSAERT, Koen, 8900 Ieper (BE)
(74) Mandataire: Boubal, Denis Henri Jacques
(86) Numéro de dépôt international: PCT/EP2009/000789
(87) Numéro de publication internationale: WO 2009/098054

(56) Documents cités:
- EP-A- 0 397 220
- WO-A-02/23990
- BE-A3- 1 016 296
- FR-A- 2 347 935
- US-A- 5 585 403
- GUIMARAES JOSE S JR ET AL: "In vitro evaluation of the disinfection efficacy on Eimeria tenella, unsporulated oocysts isolated from broilers" REVISTA BRASILEIRA DE PARASITOLOGIA VETERINARIA, vol. 16, no. 2, avril 2007 (2007-04), pages 67-71, XP002504575 ISSN: 0103-846X

## Description

La présente invention concerne l'utilisation d'une composition pour le traitement des surfaces des bâtiments d'élevage et/ou de leur matériel afin d'éliminer et/ou d'inhiber des protozoaires pathogènes et en particulier les oocystes de coccidiose.

La coccidiose est une infection provoquée par des organismes unicellulaires. Ces organismes font partie de la classe des protozoaires et on les appelle le plus souvent les oocystes. Les oocystes, dits non sporulés sont excrétés par les animaux dans les selles. Ils restent dans l'environnement et sporulent. Ces oocystes sporulés, s'ils sont ingérés, provoquent diarrhée et parfois la mort des animaux.

Dans le domaine de la lutte contre la coccidiose des animaux, il est connu d'utiliser des antibiotiques, des molécules inhibitrices du développement des oocystes : les coccidiostatiques.

Il existe peu de solutions à diluer, à appliquer dans l'environnement des animaux pour détruire des oocystes. En général, ces solutions contiennent des composés, tels que des phénols, le chloroforme et/ou l'ammoniac. Le document allemand DE-10 222 455 décrit, par exemple, l'utilisation d'un ester combiné à un phénol ayant une action contre les oocystes.

On connaît également du document BE-101 6296 l'utilisation d'alkylamine pour prévenir la prolifération des parasites des animaux et plus particulièrement les parasites suivants : *Ornithonyssus Sylvarium, Ornithonyssus Bursa, Argas* du pigeon et plus particulièrement *Dermanyssus Gallinae* (pou rouge du poulet).

On connaît également du document EP 0 397 220 un désinfectant pour l'élimination de parasites et notamment les oocystes de coccidiose, comprenant :
- un ou plusieurs désinfectant actifs appartenant au groupe des alcools aliphatiques avec 1 à 4 atomes de carbone,
- un ou plusieurs agents tensio-actif et de l'eau.
Les agents tensio-actifs préférés sont de type anioniques (RSO3-), et plus particulièrement il s'agit :
- (Formule I) d'un sulfo-ester (COOR2) d'acides gras (R1) avec R1 un acide gras végétal et R2 une chaîne C1 à C4.
- (Formule II) d'un alcool gras éthoxylé ou propoxylé, un agent tension-actif peu moussant de la famille des block copolymers avec:
   R3_O : partie alcool gras
   Si R4= H , un alcool gras éthoxylé car (CH2_CH0) est le monomère(2C)-m définissant le polymère.
   Si R4 = CH3 , un alcool gras propoxylé car (CH2-C(HO)-CH3) est le monomère (3C)- m définissant le polymère.
- (Formule III) d'un alcool gras polyéthoxylé aussi appelé polyéther alcool Eventuellement, l'agent tensio-actif peut comprendre de la fettamine et un ammonium quaternaire dans l'une de ces ramifications.

Le document FR 2.347.935 concerne un désinfectant utilisable contre les oocystes de protozoaires, et en particulier des formes résistantes de différentes espèces de coccidies. Le document FR 2.2347.935 propose le mélange suivants d'un ammonium quaternaire non classique pouvant se résumer à un dialkyl (alkyl/acyl/trialkylamino) ammonium quaternaire d'un éther phosphorique (I), un solvant aliphatique ou aromatique chloré (hydrocarbure chloré) (II) à 70_90%, d'un Alkyl di-amine tertiaire polyéthoxylée (non ionique) (III), d'un tensioactif amphotère :Alkyl (hydroxy), éventuellement d'une imidazoline (IV) et d'un agent émulsionnant anionique et /ou non ionique (émulsionnant et solubilisant des 1-3-4 dans 2) en proportion importante pour la solubilisation dans l'eau.

Cette formule est relativement non polaire avec beaucoup de solvant chloré, la dilution utilisée est de 0.25 à 10 % dans l'eau. L'imidazoline permet entre autre de garder la dilution aqueuse stable d'une formule peu polaire.

Le document FR2347935 décrit le pouvoir contre les coccidies comme une dissolution des enveloppes contenant de la kératine et des lipides. A cet effet le pouvoir du solvant chloré est mis en évidence.

Le document US 5,585,403 concerne une composition désinfectante comprenant un ammonium quaternaire combiné à un dichlorobenzène conventionnel ayant une activité contre les oocystes de coccidiose. Cette composition diluée à 1 :400 est appliquée dans l'habitat des animaux infectés. Ils 'agit d'un composé chloré ayant une action contre les coccidies.

L'article « In vitro evaluation of the disinfection efficacy on Eimeria tenella, unsporulated oocysts isolated from broilers » de GUIMARAES José daté de 2007, est basé sur une étude de l'inhibition de la sporulation de 9 désinfectants. Les résultats de cette étude montre que les désinfectants composés de sodium dodecyl benzene sulfonate , de sodium hypochlorite avec un orthodichlorobenzene et le xylène associé à des composés de glycine sont efficaces à plus de 60% sur l'inhibition de la sporulation des oocystes. Les désinfectants à base uniquement d'ammonium quaternaires (formule T1) ne semblent pas suffisamment actifs pour inhiber la sporulation des oocystes.

Les formules à base d'ammonium quaternaires testées lors de cette étude sont diluées à 1 :1000, équivalent à 0.001%, avec un temps de contact de 30 min. A titre de comparaison dans le protocole officiel allemand du DVG (et de ses directives 2000 : « *Guidelines for the testing of chemical disinfectants against coccida oocysts* »), les formules testées sont diluées à 4% avec un temps de contact de 2h.

Le protocole de test de cet article mesure la capacité des désinfectants à inhiber la sporulation des oocystes alors que le protocole DVG mesure la réduction du nombre d'oocystes après passage dans l'animal.

Le document WO 02/23990 concerne un désinfectant, comportant une amine et/ou un ammonium quaternaire pour le nettoyage et la désinfection du matériel, des instruments, des mains, des toilettes chimiques et des matériaux de construction entre autres. La désinfection comprend les activités bactéricide et fongicide.

La présente invention vise à proposer une alternative à tous les produits appliqués à l'environnement des animaux pour lutter contre la coccidiose, permettant notamment de s'intégrer parfaitement dans un programme global de sécurité.

En particulier, la composition conforme à l'invention est celle de la revendication 1 et consiste essentiellement en l'association d'une alkylamine et d'un ammonium quaternaire ou d'un dérivé d'ammonium quaternaire. Elle trouvera une application particulière pour éliminer et/ou inhiber la souche d'oocystes *Eimeria tenella* du poulet.

En appliquant cette composition sur les surfaces, telles que murs, sols et plafonds et les équipements des bâtiments infectés d'oocystes de coccidiose, la demanderesse a observé de manière surprenante une réduction de l'infection à un niveau au moins équivalent à celle constatée lors de l'utilisation de produits destinés à cette application, tels que les produits phénolés.

La présente invention sera mieux comprise à la lecture de la description suivante accompagnée des dessins en annexe parmi lesquels :
- la figure 1 est un tableau des résultats de comptages des contrôles en application du protocole allemand du DVG,
- la figure 2 est un tableau comparatif entre un désinfectant conforme à l'invention (composition 1) et un produit du marché à base de phénol,
- la figure 3 est une représentation graphique des résultats du tableau de la figure 1 ainsi que de la courbe de régression linéaire associée à ces résultats.

La présente invention concerne donc l'utilisation d'une composition pour le traitement des surfaces des bâtiments d'élevage et/ou de leur matériel afin d'éliminer et/ou d'inhiber des protozoaires pathogènes et en particulier les oocystes de coccidiose.

Selon l'invention ladite composition consiste essentiellement en l'association d'une alkylamine et d'un ammonium quaternaire ou d'un dérivé d'ammonium quaternaire.

La composition peut être appliquée par pulvérisation, thermonébulisation et/ou trempage des surfaces des bâtiments d'élevage et/ou de leur matériel.

Ladite composition peut être utilisée pour son activité sporicide et/ou kératolitique. Plus particulièrement, la composition peut contenir au moins une alkylamine conférant à la composition sa propriété kératolitique. A cet effet, l'alkylamine peut être une triamine.

L'alkylamine peut être choisie parmi le groupe suivant :la dodécylamine, l'octadécylamine, la N-suif-amine, l'oléylamine, la C₁₆₋₂₂-alkylamine, l'hexadécyldiméthylamine, la cocodiméthylamine, l'oléyldiméthylamine, la dicocométhylamine, la didécylméthylamine, la cocopropylènediamine, la C₁₆₋₂₂-alkylpropylènediamine, l'oléylpropylènediamine, la N-suif-propylènediamine, la cocopropylènediamine, l'oléyldipropylènetriamine, la N-suif-dipropylènetriamine, la N-dodécyl-dipropylènetriamine, la N-suif-dipropylènetétramine, cette liste étant non exhaustive.

Selon l'invention la composition contient un agent biocide. Plus particulièrement, cet agent biocide est un ammonium quaternaire, ou un dérivée d'ammonium quaternaire, apportant à la composition une propriété tensioactive et désinfectante.

L'ammonium quaternaire ou dérivé d'ammonium quaternaire est choisi parmi le groupe suivant :
- Composés de l'ion ammonium quaternaire, (alkyles de suif hydrogéné)triméthyles, chlorures,
- composés de l'ion ammonium quaternaire, alkyles de coco triméthyles, chlorures,
- composés de l'ion ammonium quaternaire, benzyl alkyl de coco bis(hydroxyéthyl), chlorures,
- composés de l'ion ammonium quaternaire, benzyl alkyl de coco diméthyles, chlorures,
- composés de l'ion ammonium quaternaire, dialkyles de coco diméthyles, chlorures,
- composés de l'ion ammonium quaternaire, bis(alkyl de suif hydrogéné)diméthyles, chlorures,
- composés de l'ion ammonium quaternaire, alkyl de soja triméthyles, chlorures
- composés de l'ion ammonium quaternaire, benzylalkyl en C8-18 diméthyles, chlorures,
- composés de l'ion ammonium quaternaire, benzylalkyl en C12-18 diméthyles, chlorures,
- composés de l'ion ammonium quaternaire, dialkyl en C6-12 diméthyles, chlorures,
- composés de l'ion ammonium quaternaire, benzylalkyl en C8-16 diméthyles, chlorures,
- composés de l'ion ammonium quaternaire, benzylalkyl en C12-16 diméthyles, chlorures,
- composés de l'ion ammonium quaternaire, dialkyl en C8-10 diméthyles, chlorures,
- composés de l'ion ammonium quaternaire, (oxydiéthanediyle-1,2)bis[coco alkyldiméthyl], dichlorures,
- composés de l'ion ammonium quaternaire, alkyl en C12-18 [(éthylphényl) méthyl]diméthyles, chlorures,
- composés de l'ion ammonium quaternaire, benzylalkyl en C10-16 diméthyles, chlorures,
- composés de l'ion ammonium quaternaire, benzylalkyl en C12-18 diméthyles, sels avec le dioxyde-1,1 de benzisothiazol-1,2 one-3(2H) (1:1),
- composés de l'ion ammonium quaternaire, dialkyl en C8-18 diméthyles, chlorures,
- composés de l'ion ammonium quaternaire, benzylalkyl en C12-14 diméthyles, chlorures,
- composés de l'ion ammonium quaternaire, alkyl en C12-14 [(éthylphényl) méthyl]diméthyles, chlorures,
- composés de l'ion ammonium quaternaire, benzylalkyl en C8-18 diméthyles, bromures,
- composés de l'ion ammonium quaternaire, [[[[(carboxy-2 éthyl)(hydroxy-2 éthyl) amino]-2 éthyl]amino]-2 oxo-2 éthyl]alkyl de coco diméthyles, hydroxydes, sels internes,
- polymère de N-méthylméthanamine (Einecs 204-697-4 avec(chloromethyl)oxirane (Einecs 203-439-8)/chlorure d'ammonium quaternaire polymérisé,
- iodures d'ammonium quaternaire,
- composés d'ammonium quaternaire (benzylakyldiméthyl (alkyles de C8-C22, saturés et insaturés, et alkyl de suif, alkyl de coco et alkyl de soja) chlorures, bromures ou hydroxydes/BKC
- composés d'ammonium quaternaire (dialkyldiméthyl (alkyles de C6-C18 saturés et insaturés, et alkyl de soufre, alkyl de coco et alkyl de soja) chlorures, bromures ou sulfates de méthyle/DDAC
- composés d'ammonium quaternaire (alkyltriméthyl (alkyles de C8-C18, saturés et insaturés, et alkyl de soufre, alkyl de coco et alkyl de soja) chlorures, bromures ou sulfates de méthyle/TMAC.

Selon un mode de réalisation, ladite composition peut contenir un agent tensioactif, modifiant la tension superficielle entre deux surfaces. Les agents tensioactifs sont des molécules amphiphiles présentant deux parties de polarité différentes, l'une lipophile et l'autre hydrophile et polaire. L'incorporation d'un ou plusieurs agents tensioactifs dans la composition permet de favoriser la mise en solution des ingrédients de la composition et de favoriser la mise en solution de la composition afin d'obtenir la solution prête à l'emploi. A titre d'exemple non limitatif, l'agent tensioactif est un alcool éthoxylé de type C12-C15 + 11 E.O.

Selon un mode de réalisation, la composition peut contenir un ou plusieurs agents séquestrant, ayant la propriété de former des liaisons complexes avec les ions et ainsi limiter l'effet néfaste du calcaire de l'eau servant à diluer la composition avant utilisation. A titre d'exemple non limitatif l'agent séquestrant est l'EDTA ou encore le *Nitrilo Triaceti Acid,* un excellent séquestrant du calcium de l'eau.

La présente invention peut faire partie d'un programme d'hygiène globale de l'élevage au cours duquel le bâtiment et ses équipements sont nettoyés en profondeur à l'aide d'un détergeant approprié, puis désinfectés et rincés.

La composition conforme à l'invention est ensuite appliquée, notamment à une concentration entre 4 et 20 % en poids sur toutes les surfaces du bâtiment d'élevage : sols, murs, plafonds et ses équipements.

La composition est alors appliquée à la concentration permettant une élimination des oocystes. Cette concentration peut varier d'un bâtiment à l'autre, notamment en fonction de sa configuration et de l'importance de l'infection.

A titre d'exemple, on a obtenu de bons résultats en utilisant la composition présentant la formulation en poids suivante :
- eau à 37,2 %,
- triamine à 38,4 %
- ammonium quaternaire à 5 %,
- agent tensioactif à 14,6 %
- agent séquestrant à 4,8 %.

Dans cet exemple, la triamine peut être la Dodécyl-dipropylènetriamine, le dérivée d'ammonium quaternaire peut être le Chlorure de N-(C₈-₁₈-alkyl-N-benzyl-N,N-diméthylammonium, l'agent tensioactif l' alcool éthoxylé de type C12-C15 (+ 11 E.O) et l'agent séquestrant le *Nitrilo Triaceti Acid .*

Plus généralement, la composition peut présenter une formulation en poids comprenant :
- triamine entre 20 % et 60 %,
- ammonium quaternaire entre 0 % et 10 %,
- agent tensioactif entre 0,5 % et 30 %,
- agent séquestrant entre 0 % et 10 %,
- de l'eau jusqu'à 100 %,
la somme des pourcentages faaisant 100 %.

Dans la présente invention, l'activité kératolytique de la composition a été mesurée grâce au protocole officiel allemand du DVG. Ce test a été mis en place afin d'évaluer l'efficacité des alkylamines sur les coccidies. L'alkylamine est de préférence formulée sous forme d'une solution afin de réaliser le test d'évaluation de l'efficacité décrit ci-dessous. De préférence, il s'agira d'une solution aqueuse.

Le protocole in-vivo a été réalisé en suivant les directives version 2000 du protocole allemand du DVG (*Guidelines for the testing of chemical disinfectants against coccida oocysts*).

Le pouvoir d'infection d'un inoculum d'oocystes désinfectés peut être calculé à partir de la dose d'infection et de la dose inoculée selon l'équation suivante :
- pouvoir d'infection [%] = dose infectieuse x 100 / dose inoculée
- l'efficacité du désinfectant est exprimée en % d'oocystes non infectieux : efficacité [%] = 100 - pouvoir d'infection [%].

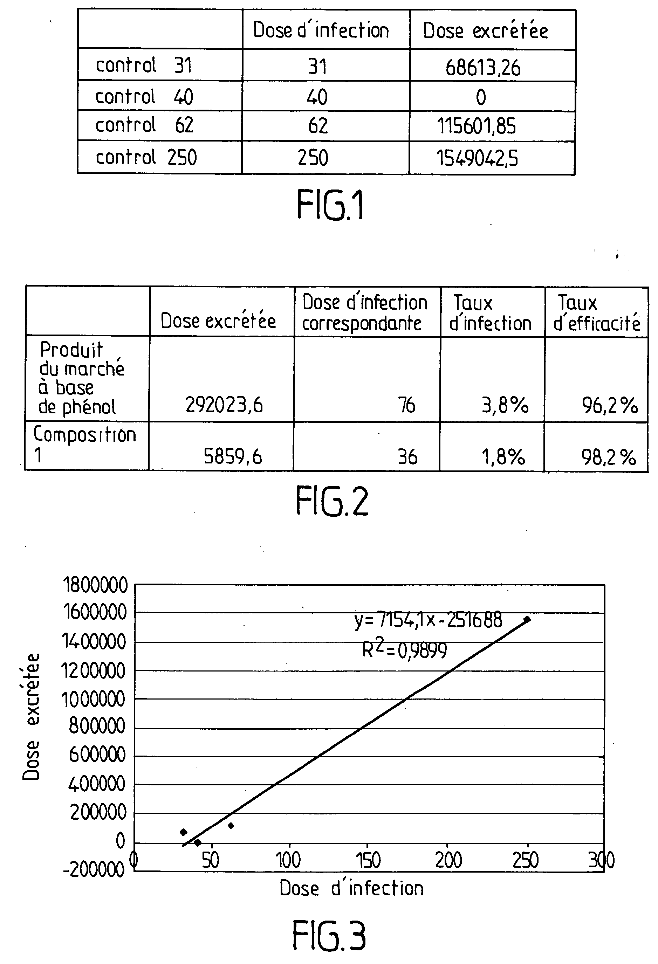

Afin de démontrer l'efficacité de la composition comme décrite dans la présente invention, la composition test, dénommée composition 1, au tableau de la figure 2, ainsi qu'un produit du marché à base de phénol, ont été testés et comparés selon le protocole présenté ci-dessus.

Les résultats sont présentés dans le tableau de la figure 1 représentant les résultats des comptages des contrôles, les résultats sont illustrés par des points dans le graphique de la figure 3 représentant la dose excrétée en fonction de la dose d'infection.

A partir de ces points, peut être calculée la courbe de régression linéaire représentant la dose excrétée en fonction de la dose d'infection.

A partir de l'équation de la courbe de régression, il est possible de déterminer la dose d'infection correspondant à l'excrétion trouvée.

Dans ce test, 2000 oocystes correspondent à un taux d'infection de 100 %. Le taux d'efficacité de ladite composition 1, est présenté au tableau de la figure 2 égale à 98,2 %. Ce taux d'efficacité est à comparer avec celui d'un produit du marché à base de phénol, à savoir 96,2 %.

Ladite composition 1 testée, à base d'alkylamine, présente donc une efficacité contre les oocystes de coccidiose selon le protocole DVG qui fixe le taux d'efficacité minimum à 95 %.

Nous décrivons par la suite quelques formulations testées permettant notamment d'illustrer l'effet de synergie entre l'alkylamine et l'ammonium quaternaire.

### Exemple 1 :

| % (p/p) | Eau | Composition A | Composition B | Composition C |
|---|---|---|---|---|
| Eau | 100 | 70 | 60 | 97,35 |
| Dodécyldipropylènetriamine | - | 30 | 40 | - |
| Chlorure de N-(C₈₋₁₈-alkyl-N-benzyl-N,N-diméthylammonium | - | - | - | 1,25 |
| Chlorure de didécyldiméthylammonium | - | - | - | 1,25 |
| % Réduction (4% - 2h) | 0 | 70,6 | 78,3 | 10,4 |

### Exemple 2 - Essais sur le type d'alkylamine

L'activité de plusieurs classes d'alkylamine a été évaluée.

On a d'abord préparé des solutions mères concentrées comprenant 30% d'alkylamine, 2,5% de chlorure de N-(C₈-C₁₈)-N-benzyl-N,N-diméthylammonium, 24% d'agents tensio-actifs amphotères, 5% de séquestrant et de l'eau en complément à 100%.

| % (p/p) | Composition D | Composition E | Composition F |
|---|---|---|---|
| C₁₆₋₂₂ alkylamine | 30 | - | - |
| oléylpropylène diamine | - | 30 | - |
| Dodécyl-dipropylène triamine | - | - | 30 |
| chlorure de N-(C₈₋₁₈-alkyl-N-benzyl-N,N-diméthylammonium | 2,5 | 2,5 | 2,5 |
| Agents tensio-actifs amphotères | 25 | 25 | 25 |
| Séquestrant | 5 | 5 | 5 |
| % Réduction (4% - 2h) | 55,3 | 47,1 | 93,5 |

### Exemple 3 : Essais sur la concentration en Alkylamine

Le tableau montre l'effet de la concentration de la dodécyldipropylènetriamine, dans un système comprenant 2,5% de chlorure de N-(C₈₋₁₈-alkyl)-N-benzyl-N,N-diméthylammonium, 24% d'agents tensio-actifs amphotères, 5% de séquestrant et de l'eau en complément à 100%.

| Concentration en dodécyldipropylènetriamine | % Réduction (4% - 2h) |
|---|---|
| 0 | 11,2 |
| 10 | 56,8 |
| 20 | 89,1 |
| 30 | 93,5 |
| 40 | 98,0 |
| 50 | Formula non dispersible |

### Exemple 4

Les essais suivants ont pour but de montrer l'avantage d'incorporer dans le composition selon l'invention un dérivé ammonium quaternaire.

Le dérivé ammonium quaternaire testé, le chlorure de N-(C₈₋₁₈-alkyl)-N-benzyl-N,N-diméthylammonium a été incorporé dans un système comprenant en poids 30% de dodécyldipropylènetriamine, 24% d'agents tensio-actifs amphotères, 5% de séquestrant et de l'eau en complément à 100%.

| Concentration en chlorure de N-(C₈₋₁₈-alkyl)-N-benzyl-N,N-diméthylammonium (%) | % Réduction (4% - 2h) |
|---|---|
| 0 | 75,3 |
| 2,5 | 93,5 |
| 5 | 95,3 |
| 10 | 98,6 |

## Revendications

1. Utilisation d'une composition pour le traitement des surfaces des bâtiments d'élevage et/ou de leur matériel afin d'éliminer et/ou d'inhiber des protozoaires pathogènes, **caractérisée en ce que** ladite composition consiste essentiellement en l'association d'une alkylamine et d'un ammonium quaternaire ou d'un dérivé d'ammonium quaternaire choisi parmi le groupe suivant :
- composés de l'ion ammonium quaternaire, (alkyles de suif hydrogéné)triméthyles, chlorures,
- composés de l'ion ammonium quaternaire, alkyles de coco triméthyles, chlorures,
- composés de l'ion ammonium quaternaire, benzyl alkyl de coco bis(hydroxyéthyl), chlorures,
- composés de l'ion ammonium quaternaire, benzyl alkyl de coco diméthyles, chlorures,
- composés de l'ion ammonium quaternaire, dialkyles de coco diméthyles, chlorures,
- composés de l'ion ammonium quaternaire, bis(alkyl de suif hydrogéné)diméthyles, chlorures,
- composés de l'ion ammonium quaternaire, alkyl de soja triméthyles, chlorures
- composés de l'ion ammonium quaternaire, benzylalkyl en C8-18 diméthyles, chlorures,
- composés de l'ion ammonium quaternaire, benzylalkyl en C12-18 diméthyles, chlorures,
- composés de l'ion ammonium quaternaire, dialkyl en C6-12 diméthyles, chlorures,
- composés de l'ion ammonium quaternaire, benzylalkyl en C8-16 diméthyles, chlorures,
- composés de l'ion ammonium quaternaire, benzylalkyl en C12-16 diméthyles, chlorures,
- composés de l'ion ammonium quaternaire, dialkyl en C8-10 diméthyles, chlorures,
- composés de l'ion ammonium quaternaire, (oxydiéthanediyle-1,2)bis[coco alkyldiméthyl], dichlorures,
- composés de l'ion ammonium quaternaire, alkyl en C12-18 [(éthylphényl) méthyl]diméthyles, chlorures,
- composés de l'ion ammonium quaternaire, benzylalkyl en C10-16 diméthyles, chlorures,
- composés de l'ion ammonium quaternaire, benzylalkyl en C12-18 diméthyles, sels avec le dioxyde-1,1 de benzisothiazol-1,2 one-3(2H) (1:1),
- composés de l'ion ammonium quaternaire, dialkyl en C8-18 diméthyles, chlorures,
- composés de l'ion ammonium quaternaire, benzylalkyl en C12-14 diméthyles, chlorures,
- composés de l'ion ammonium quaternaire, alkyl en C12-14 [(éthylphényl) méthyl]diméthyles, chlorures,
- composés de l'ion ammonium quaternaire, benzylalkyl en C8-18 diméthyles, bromures,
- composés de l'ion ammonium quaternaire, [[[[(carboxy-2 éthyl)(hydroxy-2 éthyl) amino]-2 éthyl]aminol-2 oxo-2 éthyl]alkyl de coco diméthyles, hydroxydes, sels internes,
- polymère de N-méthylméthanamine (Einecs 204-697-4 avec(chloromethyl)oxirane (Einecs 203-439-8)/chlorure d'ammonium quaternaire polymérisé,
- iodures d'ammonium quaternaire,
- composés d'ammonium quaternaire (benzylakyldiméthyl (alkyles de C8-C22, saturés et insaturés, et alkyl de suif, alkyl de coco et alkyl de soja) chlorures, bromures ou hydroxydes/BKC
- composés d'ammonium quaternaire (dialkyldiméthyl (alkyles de C6-C18 saturés et insaturés, et alkyl de soufre, alkyl de coco et alkyl de soja) chlorures, bromures ou sulfates de méthyle/DDAC
- composés d'ammonium quaternaire (alkyltriméthyl (alkyles de C8-C18, saturés et insaturés, et alkyl de soufre, alkyl de coco et alkyl de soja) chlorures, bromures ou sulfates de méthyle/TMAC.

2. Utilisation selon la revendication 1 dans lequel l'alkylamine est choisie parmi le groupe suivant : la dodécylamine, l'octadécylamine, la N-suif-amine, l'oléylamine, la C₁₆₋₂₂-alkylamine, l'hexadécyldiméthylamine, la cocodiméthylamine, l'oléyldiméthylamine, la dicocométhylamine, la didécylméthylamine, la cocopropylènediamine, la C₁₆₋₂₂-alkylpropylènediamine, l'oléylpropylènediamine, la N-suif-propylènediamine, la cocopropylènediamine, l'oléyldipropylènetriamine, la N-suif-dipropylènetriamine, la N-dodécyl-dipropylènetriamine, la N-suif-dipropylènetétramine.

3. Utilisation selon la revendication 1 ou 2, dans laquelle ladite composition est appliquée par pulvérisation, thermonébulisation et/ou trempage des surfaces des bâtiments d'élevage et de leur matériel.

4. Utilisation selon l'une quelconque des revendications 1 à 3, dans laquelle ladite alkylamine est une triamine.

5. Utilisation selon l'une quelconque des revendications 1 à 4, dans laquelle ladite composition présente un agent tensio-actif.

6. Utilisation selon l'une quelconque des revendications 1 à 5, dans laquelle ladite composition présente un agent séquestrant.

7. Utilisation selon l'une des revendications 1 à 6 dans laquelle la composition consiste en :
- une alkylamine,
- un ou plusieurs dérivés d'ammonium quaternaire,
- un agent tensioactif,
- un agent séquestrant,
- de l'eau.

8. Utilisation selon la revendication 7, dans laquelle l'alkylamine est une triamine.

9. Utilisation selon l'une quelconque des revendications 1 à 8, dans laquelle la composition présente la formulation en poids suivante :
- eau à 37,2 %,
- triamine à 38,4 %
- ammonium quaternaire à 5 %,
- agent tensioactif à 14,6 %
- agent séquestrant à 4,8 %.

10. Utilisation selon l'une quelconque des revendications 1 à 9 pour le traitement des surfaces des bâtiments d'élevage et/ou de leur matériel afin d'éliminer et/ou d'inhiber les oocystes de coccidiose.

## Patentansprüche

1. Verwendung einer Zusammensetzung zur Behandlung der Oberflächen der Zuchtgebäude und/oder ihrer Gerätschaft, um pathogene Protozoen zu beseitigen, **dadurch gekennzeichnet, dass** die Zusammensetzung im Wesentlichen in der Verbindung eines Alkylamins und eines quaternären Ammoniums oder eines quaternären Ammoniumderivats besteht, das in der folgenden Gruppe ausgewählt ist:
- Verbindungen des quaternären Ammoniumions, (hydrierte Talg-Alkyle)-Trimethyle, Chloride,
- Verbindungen des quaternären Ammoniumions, Kokos-Alkyle-Trimethyle, Chloride,
- Verbindungen des quaternären Ammoniumions, Kokos-Benzyl-Alkyl-bis(hydroxyethyl), Chloride,
- Verbindungen des quaternären Ammoniumions, Kokos-Benzyl-Alkyl-Dimethyle, Chloride,
- Verbindungen des quaternären Ammoniumions, Kokos-Dialkyl-Dimethyle, Chloride,
- Verbindungen des quaternären Ammoniumions, bis(hydriertes Talg-Alkyl)-Dimethyle, Chloride,
- Verbindungen des quaternären Ammoniumions, Soja-Alkyl-Trimethyle, Chloride,
- Verbindungen des quaternären Ammoniumions, C8-18-Benzylalkyl-Dimethyle, Chloride,
- Verbindungen des quaternären Ammoniumions, C12-18-Benzylalkyl-Dimethyle, Chloride,
- Verbindungen des quaternären Ammoniumions, C6-12-Dialkyl-Dimethyle, Chloride,
- Verbindungen des quaternären Ammoniumions, C8-16-Benzylalkyl-Dimethyle, Chloride,
- Verbindungen des quaternären Ammoniumions, C12-6-Benzylalkyl-Dimethyle, Chloride,
- Verbindungen des quaternären Ammoniumions, C6-10-Dialkyl-Dimethyle, Chloride,
- Verbindungen des quaternären Ammoniumions, (Oxydiethandiyl-1,2)bis[Kokos-Alkyldimethyl], Dichloride,
- Verbindungen des quaternären Ammoniumions, C12-18-[(Ethylphenyl)methyl]-Dimethyle, Chloride,
- Verbindungen des quaternären Ammoniumions, C10-16-Benzylalkyl-Dimethyle, Chloride,
- Verbindungen des quaternären Ammoniumions, C12-18-Benzylalkyl-Dimethyle, Salze mit Benzisothiazol-1,2 on-3(2H)-Dioxid-1,1 (1:1),
- Verbindungen des quaternären Ammoniumions, C8-18-Dialkyl-Dimethyle, Chloride,
- Verbindungen des quaternären Ammoniumions, C12-14-Benzylalkyl-Dimethyle, Chloride,
- Verbindungen des quaternären Ammoniumions, C12-14-[(Ethylphenyl)methyl]-Dimethyle, Chloride,
- Verbindungen des quaternären Ammoniumions, C8-18-Benzylalkyl-Dimethyle, Bromide,
- Verbindungen des quaternären Ammoniumions, [[[[(Carboxy-2 ethyl)(hydroxy-2 ethyl)amino]-2 ethyl]amino]-2 oxo-2 ethyl]Kokos-Alkyl-Dimethyle, Hydroxide, interne Salze,
- Polymer von N-Methylmethanamin (Einecs 204-697-4 mit (Chloromethyl)oxiran (Einecs 203-439-8)/polymerisiertes quaternäres Ammoniumchlorid,
- Iodide von quaternärem Ammonium,
- quaternäre Ammoniumverbindungen (Benzylalkyldimethyl (C8-C22-Alkyle, gesättigt und ungesättigt, und Talg-Alkyl, Kokos-Alkyl und Soja-Alkyl) Chloride, Bromide oder Hydroxide/BKC
- quaternäre Ammoniumverbindungen (Dialkyldimethyl(C-6-C18-Alkyle, gesättigt und ungesättigt, und Schwefelalkyl, Kokos-Alkyl und Soja-Alkyl) Chloride, Bromide oder Methylsulfate/DDAC,
- quaternäre Ammoniumverbindungen (Alkyltrimethyl(C8-C18-Alkyle, gesättigt und ungesättigt, und Schwefelalkyl, Kokos-Alkyl und Soja-Alkyl) Chloride, Bromide oder Methylsulfate/TMAC.

2. Verwendung nach Anspruch 1, bei der das Alkylamin in folgender Gruppe ausgewählt ist: Dodecylamin, Octadecylamin, N-Talg-Amin, Oleylamin, C₁₆₋₂₂-Alkylamin, Hexadecyldimethylamin, Kokos-Dimethylamin, Oleyldimethylamin, Di-Kokos-Methylamin, Didecylmethylamin, Kokos-Propylendiamin, C₁₆₋₂₂-Alkylpropylendiamin, Oleylpropylendiamin, N-Talg-Propylendiamin, Kokos-Propylendiamin, Oleyldipropylentriamin, N-Talg-Dipropylentriamin, N-Dodecyl-Diproylentriamin, N-Talg-Dipropylentetramin.

3. Verwendung nach Anspruch 1 oder 2, bei der die Zusammensetzung durch Zerstäubung, Heißvernebelung und/oder Eintauchen der Oberflächen der Zuchtgebäude und ihrer Gerätschaft aufgebracht wird.

4. Verwendung nach einem der Ansprüche 1 bis 3, bei der das Alkylamin ein Triamin ist.

5. Verwendung nach einem der Ansprüche 1 bis 4, bei der die Zusammensetzung ein Tensid aufweist.

6. Verwendung nach einem der Ansprüche 1 bis 5, bei der die Zusammensetzung ein Maskierungsmittel aufweist.

7. Verwendung nach einem der Ansprüche 1 bis 6, bei der die Zusammensetzung besteht in:
- einem Alkylamin,
- einem oder mehreren Derivaten von quaternärem Ammonium,
- einem Tensid,
- einem Maskierungsmittel,
- Wasser.

8. Verwendung nach Anspruch 7, bei der das Alkylamin ein Triamin ist.

9. Verwendung nach einem der Ansprüche 1 bis 8, bei der die Zusammensetzung die folgende gewichtsbezogene Formel aufweist:
- Wasser zu 37,2 %
- Triamin zu 38,4 %
- quaternäres Ammonium zu 5 %
- Tensid zu 14,6 %
- Maskierungsmittel zu 4,8 %.

10. Verwendung nach einem der Ansprüche 1 bis 9 für die Behandlung der Oberflächen der Zuchtgebäude und/oder ihrer Gerätschaft, um die Coccidiose-Oozyten zu beseitigen und/oder zu hemmen.

## Claims

1. Use of a composition for treating surfaces of livestock buildings and/or material therein so as to eliminate and/or inhibit pathogenic protozoa **characterized in that** said composition consists essentially of the combination of an alkylamine and of a quaternary ammonium or of a quaternary ammonium selected among the following group:
- compounds of the quaternary ammonium ion, (hydrogenated tallow alkyl)trimethyls, chlorides,
- compounds of the quaternary ammonium ion, coco trimethyl alkyls, chlorides,
- compounds of the quaternary ammonium ion, coco bis(hydroxyethyl) benzyl alkyl, chlorides,
- compounds of the quaternary ammonium ion, coco dimethyl benzyl alkyl, chlorides,
- compounds of the quaternary ammonium ion, coco dimethyl dialkyls, chlorides,
- compounds of the quaternary ammonium ion, bis(hydrogenated tallow alkyl)dimethyls, chlorides,
- compounds of the quaternary ammonium ion, soya trimethyl, alkyls, chlorides,
- compounds of the quaternary ammonium ion, benzyl C8-18 alkyl dimethyls, chlorides,
- compounds of the quaternary ammonium ion, benzyl C12-18 alkyl dimethyls, chlorides,
- compounds of the quaternary ammonium ion, C6-12 dialkyl dimethyls, chlorides,
- compounds of the quaternary ammonium ion, benzyl C8-16 alkyl dimethyls, chlorides,
- compounds of the quaternary ammonium ion, benzyl C12-16 alkyl dimethyls, chlorides,
- compounds of the quaternary ammonium ion, C8-10 dialkyl dimethyls, chlorides,
- compounds of the quaternary ammonium ion, (oxydiethanediyl-1,2)bis[coco alkyldimethyl], dichlorides.
- compounds of the quaternary ammonium ion C12-18 alkyl [(ethylphenyl)methyl]dimethyls, chlorides,
- compounds of the quaternary ammonium ion, benzyl C10-16 alkyl dimethyls, chlorides,
- compounds of the quaternary ammonium ion, benzyl C12-18 alkyl dimethyls, salt with benzisothiazol-1,2 one-3(2H)-1,1-dioxide, (1:1),
- compounds of the quaternary ammonium ion, C8-18 dialkyl dimethyls, chlorides,
- compounds of the quaternary ammonium ion, benzyl C12-14 alkyl dimethyls, chlorides,
- compounds of the quaternary ammonium ion, C12-14 alkyl [(ethylphenyl) methyl]dimethyls, chlorides,
- compounds of the quaternary ammonium ion, benzyl C8-18 alkyl dimethyls, bromides,
- compounds of the quaternary ammonium ion, [[[[(carboxy-2 ethyl)(hydroxy-2 ethyl) amino]-2 ethyl]amino]-2 oxo-2 ethyl]coco dimethyl alkyls, hydroxides, internal salts,
- N-methylmethanamine polymer (Einecs 204-697-4 with (chloromethyl)oxirane (Einecs 203-439-8)/ polymerised quaternary ammonium chloride,
- quaternary ammonium iodides,
- quaternary ammonium compounds (benzylalkyldimethyl (saturated and unsaturated C8-22 alkyls, and tallow alkyl, coco alkyl and soya alkyl) chlorides, bromides or Hydroxides/BKC,
- quaternary ammonium compounds (dialkyldimethyl (saturated an unsaturated (C6-18 alkyls, and sulphur alkyl, coco alkyl and soya alkyl) chlorides, bromides or methyl sulphates/DDAC, and
- quaternary ammonium compounds (alkyltrimethyl (saturated and unsaturated C8-18 alkyl, and sulphur alkyl, coco alkyl, and soya alkyl) chlorides, bromides or methyl sulphates/TMAC.

2. Use according to claim 1 wherein the alkylamine is selected from the following group: dodecylamine, octadecylamine, N-tallow-amine, oleylamine, C-16-22-alkylamine, hexadecyldimethylamine, cocodimethylamine, oleyldimethylamine, dicoco-methylamine, didecylmethylamine, cocopropylenediamine, C-16-22-alkylpropylene-diamine, oleylpropylenediamine, N-tallow-dipropylenediamine, cocopropylenediamine, oleyldipropylenetriamine, N-tallow-dipropylenetriamine, N-dodecyl-dipropylene-triamine, N-tallow-dipropylenetetramine.

3. Use according to claims 1 or 2, wherein said composition is applied by pulverisation, thermonebulisation and/or soaking of the surfaces of livestock buildings and the material therein.

4. Use according to any one of claims 1 to 3, wherein said alkylamine is a triamine.

5. Use according to any one of claims 1 to 4, wherein said composition presents a surfactant.

6. Use according to any one of claims 1 to 5, wherein said composition presents a sequestering agent.

7. Use according to any one of claims 1 to 6, wherein said composition consists of:
- an alkylamine,
- one or more quaternary ammonium derivatives,
- a surfactant,
- a sequestering agent,
- and water.

8. Use according to claim 7, wherein said alkylamine is a triamine

9. Use according to any one of claims 1 to 8, wherein said composition presents the following formulation in weight ratios:
- water 37.2%,
- triamine 38.4%,
- quaternary ammonium 5%,
- surfactant 14.6%,
- and sequestering agent 4.8%.

10. Use according to any of claims 1 to 9 for treating surfaces of livestock buildings and/or material therein so as to eliminate and/or inhibit coccidiosis oocysts.
